# EUROPEAN PATENT APPLICATION

(11) **EP 4 349 999 A1**
(43) Date of publication of application: **10.04.2024**
(21) Application number: 22815409.2
(22) Date of filing: 27.04.2022
(51) Int. Cl.: C12Q 1/02, G01N 21/01, G01N 33/48, G01N 35/00, G06T 7/20

(54) **AUTOMATED SYSTEM AND METHOD FOR MICROSCALE MONITORING OF C. ELEGANS CONTAINED IN A PETRI DISH WITH TRANSPARENT MEDIUM, MONITORING RECORD OBTAINED AND USE OF SAME**

(30) Priority: 31.05.2021 ES 202130492
(71) Applicant: Universitat Politècnica de València, 46022 Valencia (ES)
(72) Inventor: SÁNCHEZ SALMERÓN, Antonio José, 46022 Valencia (ES); PUCHALT RODRÍGUEZ, Joan Carles, 46022 Valencia (ES); GONZÁLEZ ROJO, José Felix, 46022 Valencia (ES); RICOLFE VIALA, Carlos, 46022 Valencia (ES)
(74) Representative: Ibarra Garcia, Isabel
(86) International application number: PCT/ES2022/070254
(87) International publication number: WO 2022/254058

(57) **Abstract**

The invention relates to an automated system for inspecting Petri dishes in which multiple C. *elegans* nematodes coexist, such that precise mobility characteristics of each individual C. *elegans* nematode can be extracted. The invention requires the Petri dish to be monitored to be moved in a controlled manner along the X, Y, and Z axes with respect to at least one movable camera that captures a high-resolution sequence of images for each *C. elegans* nematode, and at least one fixed camera that captures a low-resolution sequence of global images that allow the entire Petri dish to be framed. For this purpose, the movable camera is mounted on a movable head in order to move along the Z axis, enabling focusing, and the fixed camera is mounted on a movable trolley, together with the Petri dish, in order to move along the X and Y axes. The invention also relates to a method that uses the automated system.

## Description

### OBJECT OF THE INVENTION

The present invention relates to an automated system for monitoring *Caenorhabditis elegans* (*C. elegans*) contained in a Petri dish with transparent medium, the system being provided with at least one movable camera and at least one fixed camera with respect to the Petri dish for monitoring of *C. elegans* contained in said Petri dish, which cameras are positioned along the X, Y, and Z axes of a robot.

The movable camera thereby captures framed high-resolution images for each *C. elegans* nematode, and the fixed camera captures low-resolution global images of the entire Petri dish.

The presence of the movable camera and the fixed camera together with a laser pointer, lighting means and a robot controlling the position of the movable camera with respect to the dish allows each *C. elegans* nematode to be monitored and precise movement characteristics thereof over a given time period to be extracted.

The object of the invention is to provide a system that automatically establishes a multiscale tracking of multiple *C. elegans* nematodes coexisting in a Petri dish with great freedom of movement.

### BACKGROUND OF THE INVENTION

Research with *C. elegans* is a young discipline where there is a need to develop new methods and new technologies to automate assays. *C. elegans* is a nematode about 1 mm long, which is inexpensive to maintain and can be cultured in large numbers in a laboratory. *C. elegans* has many tissues and organs that are present in higher organisms, such as the nervous system, the digestive tract, the motor (muscle) system and the reproductive system, which can be easily monitored *in vivo,* using optical methods, because they are transparent throughout their entire life cycle (less than three weeks) for the wild type. Due to these characteristics, *C. elegans* has become one of the most important models in many fields of research, including aging and neurobiology.

Healthspan assays with *C. elegans* for neurodegenerative diseases are based on the search for new potential therapeutic components with which nematodes with a certain pathology are fed to monitor the evolution of their mobility patterns. At present, generally these assays are performed manually by skilled operators and are quite laborious.

The technologies being developed to detect the movement of C. *elegans* automatically are based on optical techniques.

Among the known systems that monitor C. *elegans,* the solution disclosed in patent number US2017107470 stands out. Said patent discloses wells where C. *elegans* nematodes are kept isolated so as to detect their movement over their entire lifespan. This system isolates each *C. elegans* nematode in a well having small dimensions, which alters the natural movement of the nematode, hinders the preparation of experiments, and may furthermore modify the C. *elegans* motor phenotypes.

In addition, there are known solutions in which several *C. elegans* are allowed to coexist in the same Petri dish that is several centimeters in diameter. In this sense, patent number WO2008062347 discloses a small organism locomotor recording procedure and device. The disclosed procedure detects interruptions of infrared microbeams when *C. elegans* nematodes move through the light beam. *C. elegans* nematode movements generate a transient fluctuation in the signal received by the phototransistor, and movement is detected by means of digital analysis of the phototransistor output.

The signal processing disclosed in patent number WO2008062347 is very simple, as it only detects whether or not there is movement for each phototransistor (process equivalent to fixed-threshold image segmentation).

The article *"*The Caenorhabditis elegans lifespan machine" (Stroustrup, N. et al., Nature Methods, 10(7), 665-670 (2013)) discloses a machine that scans standard dishes twice per hour and can distinguish dead *C. elegans* nematodes for lifespan assays. However, due to a low capture rate, *C. elegans* nematode movement cannot be tracked.

There are also known systems which allow a movable container to be monitored with several cameras. An example of these systems is disclosed in patent number KR20160048383A, which describes a nematode inspection system which uses several cameras and mirrors for three-dimensional monitoring of a nematode, avoiding possible concealed specimen. The objective of this patent is to enable a 3D tracking system which allows the C. *elegans* nematodes to move, such that only cameras with the same resolution are used in extracting 3D information.

In that sense, the system proposed in patent number KR20160048383A greatly limits the precision of measurements extracted during this type of monitoring, and it does not enable a microscale monitoring of each nematode, where it is necessary to adjust the focus.

Moreover, non-patent literature reference *"*Improving lifespan automation for Caenorhabditis elegans by using image processing and a post-processing adaptive data filter" (Puchalt, Joan Caries et al., Scientific Reports 10, 8729 (2020)) discloses a backlight control strategy which allows image segmentation automation during monitoring of an entire Petri dish with a fixed camera.

The mentioned reference discloses the use of different types of image processing to automate lifespan assays. In this case, the imaging processes applied depend on whether the *C. elegans* nematodes are located in the center of the dish or in an area very close to the wall of the Petri dish, where the image capture conditions are disturbed by lighting refractions. The mentioned reference also proposes a correction filter adaptive to lifespan curves.

The monitoring of an entire Petri dish with a fixed camera, the configuration of which greatly limits precision of the measurements extracted during monitoring, is also disclosed in this case. That is, the mentioned reference does not include the use of a movable camera which can offer, in combination with the images captured by the fixed camera, a microscale monitoring of each nematode either.

Moreover, non-patent literature reference *"Active backlight for automating visual monitoring:*
An analysis of a lighting control technique for Caenorhabditis elegans cultured on standard Petri plates" (Puchalt, Joan Carles. et al., PLoS One APR 16 (2019)) describes a backlight control strategy which allows image segmentation automation during the monitoring of an entire Petri dish with a fixed camera.

The main drawback of the mentioned systems resides in the fact that they work with low-resolution images, or with a limited field of view of a few millimeters. That is, the known configurations do not allow control of the process of capturing high-resolution images with complete information about the dish captured at a lower resolution at the same time (to perform global tracking).

Based on the above, the applicant of the present patent detects the need to propose a system which allows automation of the microscale monitoring of each nematode, and for this purpose includes in said configuration the combination of a fixed camera and a movable camera of different resolutions and the possibility that the Petri dish is movable along the X, Y, and Z axes with respect to the movable camera(s).

### DESCRIPTION OF THE INVENTION

The inspection system proposed below overcomes the drawbacks set forth above by proposing a system and method capable of automating the microscale extraction of characteristic of *C. elegans.*

The mentioned automation allows saving in the time to be invested in the assay and provides greater objectivity, with constant monitoring of C. *elegans* nematodes and more precise measurements.

A notable advantage of the invention is the monitoring of assays with multiple *C. elegans* nematodes where each Petri dish contains multiple *C. elegans* nematodes and the microscale extraction of mobility characteristics of each of the *C. elegans* nematodes contained in a Petri dish is possible.

That is, the present invention is capable of extracting precise mobility characteristics of multiple *C. elegans* nematodes coexisting in a Petri dish, preferably having dimensions between 150 and 35 mm in diameter. Monitoring is feasible due to the transparency of both the Petri dish and the medium. It should be noted that the main advantage of using Petri dishes having these dimensions is that they offer greater freedom of movement to *C. elegans* nematodes and furthermore allow for an increase in the number of *C. elegans* nematodes per Petri dish, thereby reducing the number of dishes needed and therefore reducing the cost of preparing the assay. By way of example, the present invention enables monitoring of multiple *C. elegans* nematodes contained in standard Petri dishes having large dimensions (for example, 55 mm in diameter) in comparison with the size of a *C. elegans* nematode (about 1 mm in length).

In this sense, the automated system of the invention requires the Petri dish to be monitored to be moved in a controlled manner along the X, Y, and Z axes with respect to at least one movable camera. In that way, an automated and robotic system is proposed, where the Petri dish and the fixed camera are mounted on a movable trolley in order to move along the X and Y axes, and the movable cameras are independently mounted on a movable head that moves along the Z axis to enable focusing.

A strategy which allows control of the process of capturing high-resolution images by the movable camera with complete information about the Petri dish captured at a lower resolution by the fixed camera is thereby proposed.

The automated system of the invention includes at least one fixed camera with respect to the Petri dish which monitors the entire dish with minimal precision (projection of the width of C. *elegans* in 3 pixels). Specifically, the fixed camera is provided with an optic that captures a low-resolution sequence of global images and allows the entire Petri dish to be framed. The purpose of the fixed camera is to provide global information about the location of all the C. *elegans* nematodes in the Petri dish, allowing control of their identities.

Moreover, the system of the present invention is provided with at least one movable camera with respect to the Petri dish which monitors a small frame around a single individual C. *elegans* nematode with maximum (microscale) precision. Specifically, the movable camera is provided with an optic that captures a high-resolution sequence of images for each C. *elegans* nematode of the Petri dish to be monitored and allows a single individual *C. elegans* nematode to be tightly framed. For this purpose, the optic of the movable camera is an optic having a focal length of between 80 mm and 135 mm.

The automated system of the invention also includes a robot which enables the movable camera to track the movement of a C. *elegans* nematode in the Petri dish while extracting the precise characteristics of its movement. The fixed camera, lighting means, and a support where the Petri dish is placed are fixed in a movable trolley that moves along the X and Y axes by means of the robot. Moreover, the movable camera and a laser pointer are mounted on a movable head that moves along the Z axis by means of the robot. That is, the robot operates the movable trolley along the X and Y axes and the movable head along the Z axis.

From the mechanical viewpoint, precision and repeatability errors are not critical because control of the robot is approached by means of visual feedback.

As previously stated, the fixed camera, the lighting means, and the support for the Petri dish are mounted together on the movable trolley that moves along the X and Y axes, with the automated system allowing two different configurations based on the type of lighting to be implemented.

In this sense, the arrangement of the lighting means is fundamental to obtaining well contrasted images, so there are defined two different lighting strategies defined by the two preferred embodiments of the system of the present invention, which will be described in detail in the following section. In that sense, the two preferred embodiments of the invention have the same configuration, differing only in the configuration of the movable trolley.

In the first preferred embodiment of the invention, referred to as lateral lighting, a fixed low-resolution camera is positioned at the base of the support, specifically below the Petri dish, which is transparent. As previously mentioned, at least one movable camera is mounted on the movable head to move it along the Z axis. That is, the movable camera captures images above the Petri dish, providing a microscale function; in this way, the robot, upon operating the movable head, allows focusing, and upon operating the movable trolley, allows varying the microscale field of view.

Alternatively, in a second preferred embodiment of the invention, referred to as backlighting, the lighting means are arranged at the base of the movable trolley on which the Petri dish rests, emitting a backlighting light beam with respect to the Petri dish. This second embodiment proposes using a light splitter to enable the possibility of using backlighting.

Like in the first preferred embodiment of the invention, in this second embodiment the movable camera mounted in the movable head works on a microscale level, moving along the Z axis, and the fixed camera works on a global level. The lighting means are coupled to the movable trolley together with the support for the Petri dish and the fixed camera.

As mentioned above, the rest of the automated system for both embodiments of the invention is the same, with active vision techniques which allow the individual microscale tracking and focusing on each *C. elegans* nematode being applied.

The system offers a global view that provides global mapping information where all C. *elegans* nematodes will be located and identified continuously. This mapping allows guiding the microscale image acquisition process, providing precise descriptors to solve posture tracking algorithms, event detection and movement pattern recognition.

Specifically, the movable camera captures a high-resolution sequence of images for each C. *elegans* nematode, which include the light emitted by the laser pointer in a position centered in each image, the high-resolution images being focused due to the position along the Z axis of the movable head with respect to the Petri dish and the high-resolution images being framed for each *C. elegans* nematode to be monitored due to the position along the X and Y axes of the movable trolley on which the support for the Petri dish is mounted .

Therefore, the laser pointer mounted on the movable head that moves along the Z axis is used to start the multiscale tracking of each *C. elegans* nematode. The laser pointer points to the field of view of the movable camera. The light emitted by the laser pointer is detected at the same time by the fixed camera.

The presence of the light emitted by the laser pointer in the image captured by the fixed camera allows control over which *C. elegans* nematode is framed with the movable camera, controlling the movement executed by the robot of the Petri dish along the X and Y axes, such that with the visual feedback from the fixed camera, the laser pointer is successfully positioned above the chosen *C. elegans* nematode for microscale monitoring.

To capture the precise information about the movement of a C. *elegans* nematode, the X, Y, and Z axes are controlled with the visual feedback from the movable camera, with the C. *elegans* nematode being centered and focused in the image as described in detail in the method used based on the system of the present invention.

XY control actions taken during a given period of time to track movement of the *C. elegans* nematode are proposed as the mobility index.

The method of the invention includes the following steps, which will be described in detail in the following section:
- constant global tracking of all the *C. elegans* nematodes with information extracted from the fixed camera;
- choosing the *C. elegans* nematode to be monitored and framing same in the movable camera, controlling the movements of the movable trolley with information extracted from the fixed camera.
- Individual mechanical tracking, where the movable camera captures a high-resolution sequence of images centered on each *C. elegans* nematode for obtaining the microscale characteristics thereof, while controlling the movements of the movable trolley and of the movable head with information extracted from the movable camera.

Specifically, to carry out the automated method of the invention, a first individual *C. elegans* nematode is chosen using the images captured by the fixed cameras of the automated system of the invention, and a framing operation is performed with the help of the laser pointer and the tracking of the individual nematode is performed with the movable cameras. The framing operation is completed when the light emitted by the laser pointer is centered on the C. *elegans* nematode chosen to be monitored.

Once the precise information about the chosen *C. elegans* nematode has been extracted, the next individual *C. elegans* nematode to be monitored is chosen, and so on until all the nematodes are monitored, such that a monitoring record for each *C. elegans* nematode is obtained.

The method described in detail above allows the monitoring of *C. elegans* based on multiple images, provided by at least one fixed camera which allows the control of the sequencing of the monitoring of all the *C. elegans* nematodes in a standard Petri dish that is several centimeters in diameter. The individual microscale images, however, are provided by at least one movable camera which allows the precise characteristics of movement during a given period of time (for example, 30 seconds) to be extracted.

Finally, it should be noted that the automated system of the invention and its automated method of monitoring allows a monitoring record to be obtained. It should be noted that, advantageously, the record obtained allows a sequence of microscale images to be provided that enables precise posture patterns of *C. elegans* nematodes to be extracted so as to determine new mobility characteristics.

Moreover, the automated system and the automated method developed are used in various applications such as: compound irritability, compound toxicity, and pharmacological tests.

Specifically, the present invention enables the automation of healthspan assays for C. *elegans,* which are used in sectors such as the pharmaceutical sector, the food sector, and the cosmetic sector.

The pharmaceutical sector uses these assays to search for new drugs that relieve the effects of neurodegenerative diseases such as Alzheimer's, Parkinsons or Huntington's. The food sector uses these assays to design new, healthier probiotic foods, adding value to foodstuffs and/or analyzing their toxicity. The cosmetics sector uses these assays to analyze the irritability or toxicity of new products.

### DESCRIPTION OF THE DRAWINGS

To complement the description that will be made below and for the purpose of helping to better understand the features of the invention according to preferred practical embodiments thereof, a set of drawings is attached as an integral part of said description, which drawings depict the following in an illustrative and non-limiting manner:
Figure 1 shows a perspective view of the automated system for microscale monitoring of C. *elegans* contained in a Petri dish with transparent medium, as described in the present invention and where neither the lighting means, the support, or the fixed camera are depicted.
Figure 2 shows a front view of the automated system depicted in Figure 1.
Figure 3 shows a perspective detail view of the movable head of the automated system depicted in the preceding figures.
Figure 4 shows a perspective detail view of the automated system according to the first preferred embodiment of the invention, where the lighting means are transverse to the support and the fixed camera is mounted on the movable trolley.
Figure 5 shows a front detail view of the automated system of the invention according to the first preferred embodiment depicted in Figure 4.
Figure 6 shows a perspective view of the automated system according to the second preferred embodiment of the invention, where the lighting means are arranged on the movable trolley and the fixed camera is arranged in such a way that it captures images from one side of the support.
Figure 7 shows a front detail view of the automated system of the invention according to the second preferred embodiment depicted in Figure 6.
Figure 8 shows a perspective view of the automated system of the invention according to the second preferred embodiment of the invention, where the path of the laser pointer is depicted.
Figure 9 shows a schematic diagram of the steps of the automated method for microscale monitoring of *C. elegans* contained in a Petri dish with transparent medium carried out with any of the preferred embodiments of the automated system of the present invention.

### PREFERRED EMBODIMENT OF THE INVENTION

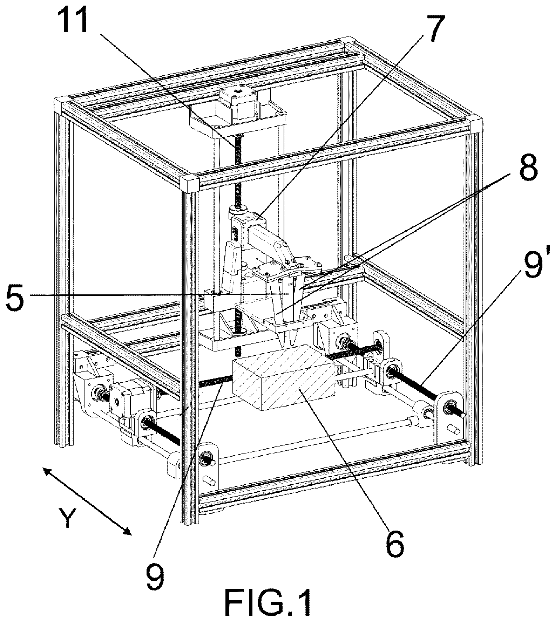

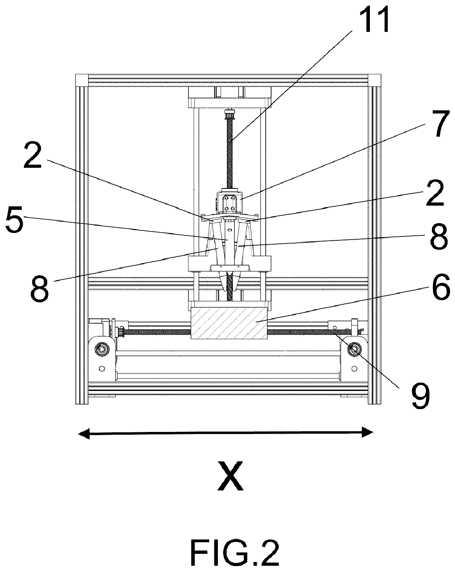

Figures 1, 2, and 3 set out in the present specification allow a depiction of some elements that are part of the automated system for microscale monitoring of *C. elegans* contained in a Petri dish with transparent medium to be observed.

Specifically, the mentioned figures depict the following elements of the automated system of the invention:
- two movable cameras (2) each one provided with an optic (8) that captures a high-resolution sequence of images for each *C. elegans* nematode of the Petri dish to be monitored and allows a single individual *C. elegans* nematode to be tightly framed;
- a laser pointer (5);
- a movable head (7) that moves along the Z axis on which the movable cameras (2) and the laser pointer (5) are mounted;
- a movable trolley (6) that moves along the X and Y axes, i.e., X axis (9) and Y axis (9'), which will contain a support (3) for the placement of the Petri dish (4) which contains the *C. elegans* to be monitored in transparent medium.

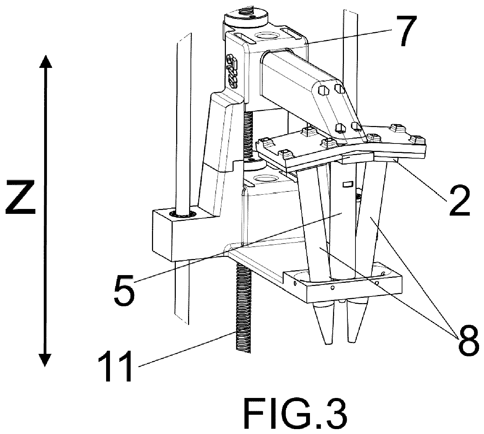

As depicted in Figure 3, the two movable cameras (2) and the laser pointer (5) are mounted on the movable head (7) such that the two movable cameras (2) are positioned with respect to the laser pointer (5) in such a way that all the images taken by the movable cameras (2) contain the light emitted by the laser pointer (5) in a centered position.

The automated system of the invention is provided with a robot that operates the movable trolley (6) along the X and Y axes (9) and (9') and the movable head (7) along the Z axis (11), enabling the movement of the movable cameras (2) along the X, Y, and Z axes with respect to the Petri dish.

The automated system of the present invention includes a fixed camera (1) and lighting means which are not depicted in Figures 1, 2, and 3, since the two preferred embodiments of the invention described in detail below materialize depending on their location.

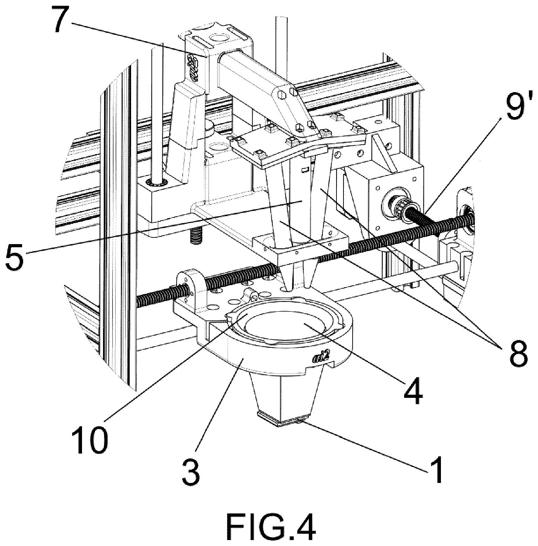

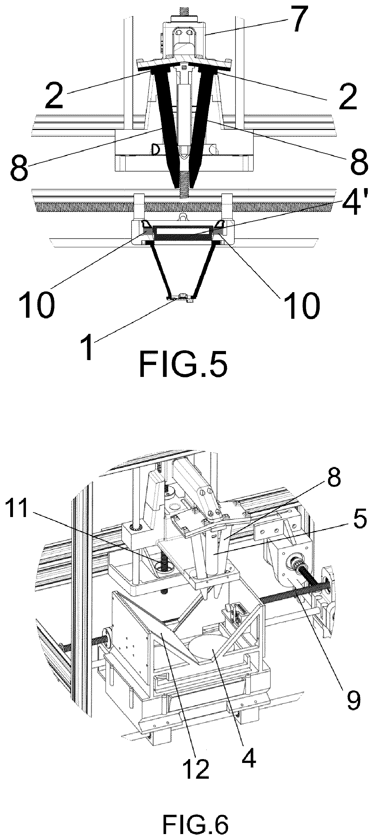

Figures 4 and 5 specifically depict the automated system according to the first preferred embodiment of the invention. Figures 6, 7, and 8, however, correspond to depictions of the automated system of the present invention according to the second preferred embodiment.

In both preferred embodiments of the invention, the fixed camera (1) is provided with an optic that captures a low-resolution sequence of global images and allows the entire Petri dish (4) to be framed, with the Petri dish (4) being lit by the lighting means. Preferably, the lighting means are cold and homogeneous and made up of LEDs to project homogeneous lighting onto the Petri dish (4) to be monitored.

Moreover, also in both preferred embodiments of the invention, the laser pointer (5) emits a light which allows the framing of the movable camera (2) to be located with respect to the image captured by the fixed camera (1). The movable camera (2) thereby captures a high-resolution sequence of images for each *C. elegans* nematode, which include the light emitted by the laser pointer (5) in a position centered in each image, the high-resolution images being focused due to the position along the Z axis of the movable head (7) with respect to the Petri dish (4), and the high-resolution images being framed for each *C. elegans* nematode to be monitored due to the position along the X and Y axes (9) and (9') of the movable trolley (6) on which the support (3) for the Petri dish (4) is mounted .

In that sense, in the first preferred embodiment of the invention depicted in Figures 4 and 5 it can be seen that the fixed camera (1) is mounted on the movable trolley (6) which is provided with a support (3). The fixed camera (1) thereby captures images from an angle opposite the movable camera (2), with the lighting means (10) being transverse with respect to the Petri dish (4), being arranged in such a way that they emit a transverse ring-shaped light beam in the support (3). Preferably, the lighting means (10) in the first preferred embodiment of the invention are centered at the height of the surface of the transparent medium of the Petri dish (4) to offer more homogeneous lighting of the Petri dish (4).

Moreover, in the second preferred embodiment of the invention depicted in Figures 6, 7, and 8 it can be seen that the fixed camera (1') is arranged in such a way that it captures images from one side of the support (3) and the lighting means (10') are arranged at the base of the movable trolley (6). In that sense, the lighting means (10') emit a backlighting light beam with respect to the Petri dish (4). The automated system is provided with a beam splitter (12).

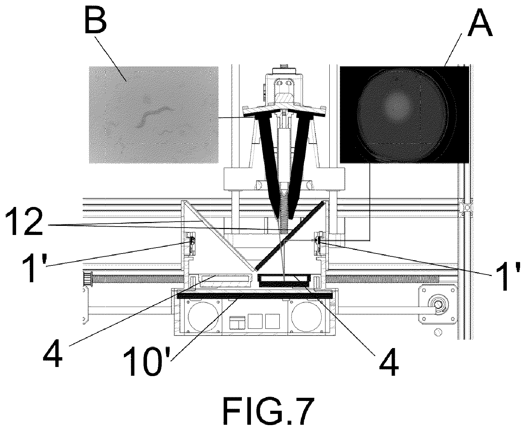

Advantageously, the second embodiment of the invention has a configuration in which the inclusion of two fixed cameras (1') arranged in alignment along the X axis (9) and facing one another is foreseen, as observed in Figure 7. In this configuration, the support (3) is provided with two housings arranged along the X axis (9) for the placement of two Petri dishes (4). The monitoring of two Petri dishes (4) is thereby enabled, since movement along the X and Y axes (9) and (9') of the movable trolley (6) which contains the support (3) with two Petri dishes (4) allows the mobility of all the *C. elegans* nematodes contained in both Petri dishes (4) to be monitored.

Figure 7 includes an example of images captured by the fixed camera (1'), image A, and the movable camera (2), image B. In that sense, it can be seen that image A captures a global view of the Petri dish (4) while image B shows the individual *C. elegans* nematode selected for monitoring.

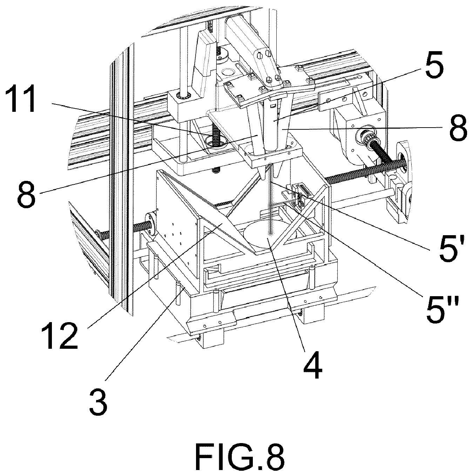

As observed in Figure 8, in the second preferred embodiment of the invention, the backlighting light beam orthogonally goes through the Petri dish (4) such that a part of the light goes through the light splitter towards the movable camera (2) (located at the top in the movable head (7)) and another part of the light beam is reflected and strikes the fixed camera (1'). Preferably, the beam splitter (12) is arranged at 45º, as depicted in Figure 7, such that the part of the light beam that is reflected and strikes the fixed camera (1') does so by being reflected at 45°.

As described in detail in the description section, the automated method used in any of the preferred embodiments of the system of the invention follows steps that allow precise mobility information to be extracted from all the *C. elegans* nematodes contained in a Petri dish.

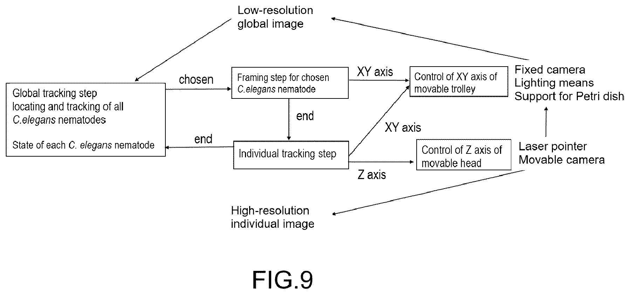

Specifically, the steps of the method of the invention can be seen schematically in Figure 9 and are the following:
- global tracking of all the *C. elegans* nematodes with information extracted from the fixed camera (1). This step is executed cyclically in an uninterrupted manner such that each time the fixed camera (1) captures a global image, the system locates all the *C. elegans* nematodes in the Petri dish (4). This step comprises the following sub-steps:
   ∘ capturing a low-resolution sequence of global images of the entire Petri dish (4) by the fixed camera (1) for the uninterrupted locating of all the C. *elegans* nematodes contained in the Petri dish (4);
   ∘ processing each global image captured for the uninterrupted locating of the position of all the *C. elegans* nematodes;
- choosing the *C. elegans* nematode to be monitored and framing same in the movable camera (2), controlling the movements of the movable trolley (6) with information extracted from the fixed camera (1). The framing step is executed until the light emitted by the laser pointer (5) is centered on the next *C. elegans* nematode to be monitored. This step comprises the following sub-steps:
   ∘ choosing a C. *elegans* nematode to be monitored, or the end of the process if all the *C. elegans* nematodes contained in the Petri dish (4) have already been monitored;
   ∘ processing each image captured by the fixed camera (1) for the uninterrupted locating of the position of the laser pointer (5);
   ∘ a control system calculates the control actions for controlling the movements to be applied on the movable trolley (6) along the X and Y axes (9) and (9') to cancel out the error between the position of the laser pointer (5) and the position of the chosen *C. elegans* nematode;
   ∘ the robot executes the control actions for controlling movement of the movable trolley (6) along the X and Y axes (9) and (9') by matching the position of the laser pointer (5) with the position of the chosen *C. elegans* nematode in the low-resolution image, at which time the movable camera (2) will have the chosen *C. elegans* nematode within its field of view or framing;

- individual tracking where the movable camera (2) captures a high-resolution sequence of images centered on each *C. elegans* nematode for obtaining the microscale characteristics thereof, while controlling the movements of the movable trolley (6) and of the movable head (7) with information extracted from the movable camera (2). The step is executed until all the necessary information about the movement characteristics of the chosen *C. elegans* nematode has been extracted.

This step comprises the following sub-steps:
∘ tracking the movements of the *C. elegans* nematode, which in turn comprises:
   ▪ processing each image captured by the movable camera (2) for the uninterrupted locating of the position of the *C. elegans* nematode;
   ▪ a control system calculates the control actions for controlling the movements to be applied on the movable trolley (6) along the X and Y axes (9) and (9') to cancel out the error between the position of the *C. elegans* nematode and the center of the high-resolution image;
   ▪ the robot executes the control actions for controlling movement of the movable trolley (6) along the X and Y axes (9) and (9'), keeping the C. *elegans* nematode centered in the high-resolution images;
∘ focusing the image captured by the movable camera (2) by means of the movement of the movable camera (2) along the Z axis (11);
∘ extracting and recording a high-resolution sequence of images captured by the movable camera (2) while maintaining the two previous steps until obtaining the necessary images in which the chosen *C. elegans* nematode appears in a centered manner so as to obtain the microscale characteristics thereof, ending the monitoring of the chosen *C. elegans* nematode;

∘ choosing and framing a new *C. elegans* nematode to be monitored.

It should be noted that the global tracking step is maintained in parallel to the step of choosing the *C. elegans* nematode to be monitored and framing same and the step of individual tracking.

That is, when the step of choosing the *C. elegans* nematode to be monitored and framing same ends, the step of individual tracking begins and is also executed in parallel with the global tracking step.

## Claims

1. An automated system for microscale monitoring of *C. elegans* contained in a Petri dish with transparent medium, **characterized in that** it comprises:
- at least one fixed camera (1) provided with an optic that captures a low-resolution sequence of global images and allows the entire Petri dish (4) to be framed;
- at least one movable camera (2) provided with an optic (8) that captures a high-resolution sequence of images for each *C. elegans* nematode of the Petri dish (4) to be monitored and allows a single individual *C. elegans* nematode to be tightly framed;
- lighting means;
- a support (3) for the placement of at least one Petri dish (4);
- a laser pointer (5) the emitted light of which allows the framing of the movable camera (2) to be located with respect to the image captured by the fixed camera (1);
- a movable trolley (6) that moves along the X and Y axes (9) and (9') on which at least the support (3) and the fixed camera (1) are mounted;
- a movable head (7) that moves along the Z axis on which the movable camera (2) and the laser pointer (5) are mounted;
- a robot that operates the movable trolley (6) along the X and Y axes (9) and (9') and the movable head (7) along the Z axis (11);
**characterized in that** the movable camera (2) captures a high-resolution sequence of images for each *C. elegans* nematode, which include the light emitted by the laser pointer (5) in a position centered in each image, the high-resolution images being focused due to the position along the Z axis of the movable head (2) with respect to the Petri dish (4) and the high-resolution images being framed for each *C. elegans* nematode to be monitored due to the position along the X and Y axes of the movable trolley (6) on which the support (3) for the Petri dish (4) is mounted .

2. The automated system for microscale monitoring of C. *elegans* contained in a Petri dish with transparent medium according to claim 1, **characterized in that** it includes two movable cameras (2) positioned with respect to the laser pointer (5) in such a way that all the images taken by the movable cameras (2) contain the light emitted by the laser pointer (5) in a centered position.

3. The automated system for microscale monitoring of *C. elegans* contained in a Petri dish with transparent medium according to claim 1, **characterized in that** the Petri dish (4) has dimensions of between 150 and 35 mm in diameter.

4. The automated system for microscale monitoring of *C. elegans* contained in a Petri dish with transparent medium according to claim 1, **characterized in that** the lighting means are cold and homogeneous and made up of LEDs.

5. The automated system for microscale monitoring of C. *elegans* contained in a Petri dish with transparent medium according to claim 1 or 2, **characterized in that** the fixed camera (1) is mounted on the movable trolley (6) capturing images from an angle opposite the movable camera (2) and the lighting means (10) are transverse with respect to the Petri dish (4), being arranged in such a way that they emit a ring-shaped light beam onto the support (3).

6. The automated system for microscale monitoring of C. *elegans* contained in a Petri dish with transparent medium according to claim 3, **characterized in that** the lighting means (10) are centered at the height of the surface of the transparent medium of the Petri dish (4).

7. The automated system for microscale monitoring of C. *elegans* contained in a Petri dish with transparent medium according to claim 1 or 2, **characterized in that** the fixed camera (1) is arranged such that it captures images from one side of the support (3) and the lighting means (10') are arranged at the base of the movable trolley (6), emitting a backlighting light beam with respect to the Petri dish (4), the automated system being provided with a beam splitter (12).

8. The automated system for microscale monitoring of C. *elegans* contained in a Petri dish with transparent medium according to claim 4 or 5, **characterized in that** it is provided with two fixed cameras (1') arranged in alignment along the X axis (9) and facing one another; and the support (3) is provided with two housings arranged along the X axis (9) for the placement of two Petri dishes (4).

9. An automated method for microscale monitoring of *C. elegans* contained in a Petri dish with transparent medium using the system according to any of the preceding claims, **characterized in that** it simultaneously performs global tracking and individual tracking and comprises the following steps:
- global tracking of all the *C. elegans* nematodes with information extracted from the fixed camera (1), which comprises:
∘ capturing a low-resolution sequence of global images of the entire Petri dish (4) by the fixed camera (1) for the uninterrupted locating of all the C. *elegans* nematodes contained in the Petri dish (4);
∘ processing each global image captured for the uninterrupted locating of the position of all the *C. elegans* nematodes;
- choosing the *C. elegans* nematode to be monitored and framing same in the movable camera, controlling the movements of the movable trolley with information extracted from the fixed camera (1), which comprises:
∘ choosing a C. *elegans* nematode to be monitored, or the end of the process if all the *C. elegans* nematodes contained in the Petri dish (4) have already been monitored;
∘ processing each image captured by the fixed camera (1) for the uninterrupted locating of the position of the laser pointer (5);
∘ a control system calculates the control actions for controlling the movements to be applied on the movable trolley (6) along the X and Y axes to cancel out the error between the position of the laser pointer (5) and the position of the chosen *C. elegans* nematode;
∘ the robot executes the control actions for controlling movement of the movable trolley (6) along the X and Y axes by matching the position of the laser pointer (5) with the position of the chosen *C. elegans* nematode in the low-resolution image, at which time the movable camera (2) will have the chosen *C. elegans* nematode within its field of view or framing;
- individual tracking, where the movable camera (2) captures a high-resolution sequence of images centered on each *C. elegans* nematode for obtaining the microscale characteristics thereof, while controlling the movements of the movable trolley (6) and of the movable head (7) with information extracted from the movable camera (2), which comprises:
∘ tracking the movements of the *C. elegans* nematode, which in turn comprises:
▪ processing each image captured by the movable camera (2) for the uninterrupted locating of the position of the *C. elegans* nematode;
▪ a control system calculates the control actions for controlling the movements to be applied on the movable trolley (6) along the X and Y axes (9) and (9') to cancel out the error between the position of the *C. elegans* nematode and the center of the high-resolution image;
▪ the robot executes the control actions for controlling movement of the movable trolley (6) along the X and Y axes, keeping the C. *elegans* nematode centered in the high-resolution images;
∘ focusing the image captured by the movable camera (2) by means of the movement of the movable camera (2) along the Z axis (11);
∘ extracting and recording a high-resolution sequence of images captured by the movable camera (2) while maintaining the two previous steps until obtaining the necessary images in which the chosen *C. elegans* nematode appears in centered manner so as to obtain the microscale characteristics thereof, ending the monitoring of the chosen *C. elegans* nematode;
∘ choosing and framing a new *C. elegans* nematode to be monitored.

10. A monitoring record obtained by the method according to claim 9.

11. Use of the method according to claim 9 or the system according to one of claims 1 to 8, for use thereof selected from: compound irritability, compound toxicity, and pharmacological tests.
